(19) Europäisches Patentamt / European Patent Office / Office européen des brevets

(11) **EP 4 144 289 A1**

(12) **EUROPEAN PATENT APPLICATION**

(43) Date of publication:
08.03.2023 Bulletin 2023/10

(21) Application number: 21194655.3

(22) Date of filing: 02.09.2021

(51) International Patent Classification (IPC):
*A61B 5/00* $^{(2006.01)}$    *A61B 5/021* $^{(2006.01)}$
*A61B 5/024* $^{(2006.01)}$    *A61B 5/0285* $^{(2006.01)}$
*A61B 5/11* $^{(2006.01)}$

(52) Cooperative Patent Classification (CPC):
A61B 5/0064; A61B 5/0077; A61B 5/02125;
A61B 5/02416; A61B 5/0285; A61B 5/1102;
A61B 5/1126; A61B 5/6889; A61B 5/70

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Koninklijke Philips N.V.
5656 AG Eindhoven (NL)

(72) Inventors:
• VAN GASTEL, Mark Josephus Henricus
5656 AE Eindhoven (NL)
• VERKRUIJSSE, Willem
5656 AE Eindhoven (NL)
• MENA BENITO, Maria Estrella
5656 AE Eindhoven (NL)

(74) Representative: Philips Intellectual Property &
Standards
High Tech Campus 52
5656 AG Eindhoven (NL)

(54) **SYSTEM AND METHOD FOR DETERMINING A PHYSIOLOGICAL PARAMETER OF A SUBJECT**

(57)     A system (100) for determining a physiological parameter (250) of a subject (102) comprises first and second radiation sources (110, 120), first and second detectors (130, 140), and a processor (150). The first radiation source (110) emits a laser radiation (112) towards a first region (104) of the subject (102) proximal to a heart (105) of the subject (102). The second radiation source (120) emits an electromagnetic radiation (122) towards a second region (106) of the subject (102) remote from the first region (104) of the subject (102). The first detector (130) acquires a first image data (210) from a laser speckle pattern (212) generated at the first region (104). The second detector (140) acquires a second image data (220) from a subject radiation (222) reflected from or transmitted through a skin (108) at the second region (106). The processor (150) extracts an SV-signal (214) from the first image data (210), a PPG-signal (224) from the second image data (220), and determines a PTT (230) based on the SV-signal (214) and the PPG-signal (224).

FIG. 1

**Description**

FIELD OF THE INVENTION

The present invention relates to a system for determining a physiological parameter of a subject, such as a person or an animal. The present invention further relates to a corresponding method and a computer program product for carrying out the method.

BACKGROUND OF THE INVENTION

Physiological parameters of a subject, for example, a heart rate (HR), a respiration rate (RR) or an arterial blood oxygen saturation, may serve as indicators of a current state of the subject, and may also predict serious medical events. Such physiological parameters may further enable evaluation of a health of the subject. Therefore, the physiological parameters are extensively monitored in inpatient and outpatient care settings, at home or in further health, leisure and/or fitness settings.

Some conventional systems and methods for determining the physiological parameters may be invasive and use catheters. The conventional systems and methods that are invasive may not be recommended for healthy subjects in their daily lives as well as for the subjects receiving non-intensive care.

A pulse transit time (PTT) is generally used for determining various physiological parameters. The PTT may be measured from arrival times of a cardio-vascular pressure wave (CPW) at different anatomical locations, i.e., from an arrival time of the CPW at a location proximal to a heart and from an arrival time of the CPW at a location distal to the heart.

In some conventional systems and methods, the arrival times of the CPW at the location proximal to the heart and at the location distal to the heart may be determined using photoplethysmography (PPG). In some conventional systems and methods, the arrival times of the CPW at the location proximal to the heart and at the location distal to the heart may be determined using speckle patterns.

In some other conventional systems and methods, the physiological parameters may be determined using electrocardiography (ECG) and PPG. ECG may require various sensors and probes to be applied on a skin of the subject, e.g., on a chest of the subject. Further, the sensors and probes may require a wired/wireless communication for transmitting obtained ECG signals to a processor in order to measure the PTT. Thus, ECG may be manual, cumbersome, and/or time consuming. In addition, ECG may not accurately measure the moment of blood ejection or the start of the CPW from the heart. ECG uses R-peak as the arrival time reference of the CPW at the location proximal to the heart. However, the R-peak comprises a pre-ejection period (PEP). Therefore, the conventional systems and methods which uses ECG to measure the arrival time of the CPW at the location proximal to the heart and PPG to measure the arrival time of the CPW at the location distal to the heart, measure a pulse arrival time (PAT) instead of PTT, where PAT = PEP + PTT. Since the PAT may be variable up to tens of milliseconds, may vary from subject to subject, and may vary from time to time for the same subject, the PAT measured using ECG may not be accurate and reliable, for example, as described in the paper of Muehlsteff et al., "Cuffless estimation of systolic blood pressure for short effort bicycle tests: the prominent role of the pre-ejection period", 2006 International Conference of IEEE engineering and medicine.

Verkruysse et al., "Remote plethysmographic imaging using ambient light", Optics Express, 16(26), 22 December 2008, pp. 21434-21445 demonstrates that PPG signals can be measured remotely using ambient light and a conventional consumer level video camera, using red, green, and blue (RGB) color channels.

The PPG measurements generally require an exposed skin in order to detect subtle color variations on the skin by the video camera which are caused by pulsatile blood volume variations. However, the location proximal to the heart may be covered by a clothing and/or a blanket in healthcare settings. Further, not all exposed skin regions can be used for the PPG measurements. This may be due to varying density and depth of a cardiovascular bed of the tissue. Therefore, some exposed skin locations may provide a much weaker or even an absent PPG signal. In some cases, a phase delay of the PPG signals obtained at different locations may be large and therefore not suitable for the PTT measurements. In some other cases, skin geometry and pigmentation may vary from subject to subject and from location to location for the same subject, and may affect the accuracy of the PTT.

Golberg et al., "Large-scale clinical validation of noncontact and continuous extraction of blood pressure via multipoint defocused photonic imaging", Vol. 57, No. 7 / March 1, 2018 / Applied Optics, proposed a remote optical system based on temporal analysis of secondary reflected speckle patterns for computing a subject's PTT from the delay between the chest and wrist. However, speckle patterns at the locations distal from the heart may be representative of mechanical waves induced due to the CPW as well as mechanical waves which are induced by the heart and transmitted by a body or a skeleton of the subject. Further, differentiating between the two types of mechanical waves at the locations distal to the heart may be difficult as they may share the same periodicity.

Therefore, conventional approaches may have shortcomings which are of practical nature (contact probes, wires, viewing angle, installation, etc.), as well as of a fundamental nature (timing accuracy, signal source, etc.). Thus, the conventional systems and methods may not be optimal for determining the physiological parameters and may even cause discomfort to the subject.

WO 2015/078735 A1 discloses a device and method for obtaining pulse transit time and/or pulse wave velocity information of a subject. Based on a set of image frames of a subject and detected motion of body parts of the subject, regions of interest are selected from different non-moving body parts and pulse transit time and/or pulse wave velocity information is obtained from acquired PPG signals extracted from different regions of interest and the respective determined physical distance between the respective regions of interest. As described above, it may be impractical or difficult to implement PPG at some locations. Further, using PPG at some locations may yield inaccurate results.

SUMMARY OF THE INVENTION

As noted above, there are a number of disadvantages associated with the currently available techniques for determining a physiological parameter of a subject. For example, some of the currently available techniques may be invasive or require placing components, such as contact probes on a skin of the subject. Further, some of the currently available techniques may be inaccurate and unreliable.

It is an object of the present invention to provide improved systems and methods for determining the physiological parameter of the subject. In particular, it is an object of the present invention to provide systems and methods for unobtrusively, accurately, reliably, and efficiently determining the physiological parameter of the subject. The systems and methods of the present invention may be easily and practically implemented with minimum inconvenience to the subject. The systems and methods of the present invention may also allow contactless measurement without the need for contact probes and associated wired/wireless connections.

To better address one or more of the concerns mentioned earlier, in a first aspect of the present invention, a system for determining a physiological parameter of a subject is provided. The system comprises a first radiation source, a second radiation source, a first detector, a second detector, and a processor. The first radiation source is configured to emit a laser radiation towards a first region of the subject proximal to the heart of the subject. The second radiation source is configured to emit an electromagnetic radiation towards a second region of the subject remote from the first region of the subject. The first detector is configured to acquire a first image data from a laser speckle pattern generated at the first region in response to irradiation by the laser radiation from the first radiation source. The second detector is configured to acquire a second image data from a subject radiation reflected from or transmitted through a skin at the second region in response to irradiation by the electromagnetic radiation from the second radiation source. The processor processes the first image data and the second image data. The processor is communicably coupled to the first detector and the second detector. The processor is configured to extract a speckle vibrometry signal (SV-signal) from the first image data; extract a photoplethysmography signal (PPG-signal) from the second image data; and determine a pulse transit time (PTT) based on the SV-signal and the PPG-signal.

The present invention provides an improved system for determining the physiological parameter of the subject with an increased accuracy. As discussed above, various conventional systems for determining the physiological parameter or the PTT have their respective disadvantages. The inventors have found that a combination of SV and PPG techniques in order to determine the PTT may eliminate or mitigate the respective disadvantages of the individual techniques while leveraging the unique properties of both the techniques. Specifically, the SV and PPG techniques are used at the first region (proximal to the heart) and the second region (distal to the heart), respectively.

The system of the present invention may allow a completely contactless and continuous measurement of the physiological parameter. Therefore, the system may be suitable for a subject with extreme skin sensitivity, such as a Neonatal Intensive Care Unit (NICU) patient with extremely fragile skin, a premature baby, or a patient with extensive burns, who may require constant monitoring of the physiological parameter.

The PTT may be a valuable parameter for determining the physiological parameter. The physiological parameter may provide a reliable assessment of the cardiovascular system of the subject and/or for respiratory sleep monitoring of the subject.

The first region may be in a chest, a torso, or a back of the subject. The first region may be in the chest or the torso when the patient is in a supine position. The first region may be in the back when the patient is in a prone position. In some cases, the first region may be in a left side of the chest of the patient when the patient is in a left lateral recumbent position or in a right side of the chest of the patient when the patient is in a right lateral recumbent position. Therefore, the first region of the subject is usually covered (e.g., with a clothing and/or a blanket), specifically in a clinical or healthcare setting. The first detector may detect variations in the laser speckle pattern generated at the first region even when the first region is covered. Further, the PTT based on the SV-signal may provide a more accurate measurement of the PTT. Further, the PTT may determine the physiological parameter more accurately than a PAT measured using ECG which may be variable up to tens of milliseconds, may vary from subject to subject, and may vary from time to time for the same subject. Further, the second region may be selected from a location which usually has an exposed skin and provides a strong PPG-signal. For example, the second region may be a portion of a head or of a limb (e.g., a wrist or a finger) of the subject. Further, the PPG-signal may not be susceptible to mechanical waves transmitted through a body or a skeleton of the subject.

According to an embodiment, the processor may be further configured to determine a first time indicative of a systolic event at the first region based on the SV-signal; determine a second time indicative of an arrival of a cardiac pressure wave at the second region based on the PPG-signal; and determine the PTT based on a difference between the second time and the first time. The first time indicative of the systolic event at the first region may be determined more accurately based on the SV-signal than from a R-peak of an ECG-signal. Further, the first time indicative of the systolic event at the first region based on the SV-signal may not comprise substantial delays as in other conventional techniques. Further, the second time indicative of the arrival of the cardiac pressure wave at the second region based on the PPG-signal may also be accurately determined as the arrival of the cardiac pressure wave at the second region may be directly determined from the PPG-signal. Therefore, the PTT based on the difference between the second time and the first time may be accurate and reliable.

Optionally, the processor may be further configured to determine a pulse wave velocity (PWV) based on the PTT and a physical distance between the first region and the second region. The physical distance can be determined by using a two-dimensional (2D) camera to obtain a 2D image of at least a portion of the subject including the first and second regions. The physical distance can be determined from the 2D image by various methods, such as applying pinhole projection model on the 2D image; in case the 2D camera is calibrated, directly translating a pixel distance between the first and second regions in the 2D image into the physical distance; and in case the 2D camera is not calibrated, translating the pixel distance between the first and second regions in the 2D image into the physical distance based on an object (e.g., a ruler, a pattern, etc.) in the 2D image with known dimensions.

Optionally, the processor may be further configured to determine the physiological parameter based, at least in part, on the PWV or the PTT. Some physiological parameters may be determined based on the PWV. Some physiological parameters may be determined based on the PTT. In some cases, determination of a particular physiological parameter (e.g., blood pressure) based on the PWV may be more accurate as compared to the determination of the particular physiological parameter based on the PTT.

According to an embodiment, the first detector may be a camera. In some embodiments, the system may be configured to adjust a focus of the camera to acquire the first image data from the laser speckle pattern. The system may adjust the focus of the camera based on the requirements of the system, such as a field of view of the first detector, minimal resolution and signal-to-noise requirements, as well as a distance of the first detector from the first region.

Optionally, the system may further comprise an overview camera communicably coupled to the system and configured to generate an image of the subject. The image generated by the overview camera may determine the physical distance between the first region and the second region. The physical distance may be used to determine the PWV.

Optionally, the system may be further configured to adjust a position of the first detector and/or a position of the second detector based on the image of the subject. Therefore, the adjustment of the position of the first detector and/or the position of the second detector may be done automatically based on the image of the subject. Automatic adjustment may be required in various situations, for example, in case of any changes to the first and second regions.

Optionally, the system may be further configured to determine a position of the subject based on the image of the subject; and adjust the position of the first detector and/or the position of the second detector based, at least in part, on the position of the subject. Therefore, the system may be able to determine the physiological parameter of the subject even when the subject is in different positions. For example, in some settings, the physiological parameter of the subject may be determined while the subject is sitting. In some settings, the physiological parameter of the subject may be determined while the subject is standing. In some cases, the system may automatically adjust the position of the first detector and/or the position of the second detector if the position of the subject changes from a first position (e.g., lying position) to a second position (e.g., sitting position).

Optionally, the system may be further configured to determine a location of the first region and a location of the second region based on the image of the subject; adjust the position of the first detector based on the location of the first region; and adjust the position of the second detector based on the location of the second region. Therefore, the system may determine the physiological parameter of multiple subjects having different heights. In some cases, the system may distinguish one subject from another subject based on artificial intelligence (AI) processing. The adjustment of the positions of the first and second detectors based on the respective locations of the first and second regions may ensure that the PTT is determined accurately.

Optionally, the system may be further configured to adjust a position of the first radiation source and/or a position of the second radiation source based on the image of the subject. This may further ensure the first and second regions receive the laser radiation and the electromagnetic radiation, respectively.

Optionally, the system may further comprise at least one movable mirror configured to guide a radiation from the subject to the first detector and/or the second detector. In these embodiments, the system may be further configured to adjust a position of the at least one movable mirror based on the image of the subject. In some cases, for example, when the subject may be in a large bed or far from the first detector and/or the second detector, the adjustment of the first detector and/or the second detector may not be sufficient to ensure that the first detector and/or the second detector receive the radiation from subject. In such cases, the mirror may enhance the field of operation of the system, thereby enabling the first detector and/or the second detector to receive the radiation from the subject.

In a second aspect, a method for determining a physiological parameter of a subject is provided. The method comprises emitting a laser radiation towards a first region of the subject proximal to a heart of the subject; emitting an electromagnetic radiation towards a second region of the subject remote from the first region of the subject; acquiring a first image data from a laser speckle pattern generated at the first region in response to irradiation by the laser radiation; acquiring a second image data from a subject radiation reflected from or transmitted through a skin at the second region in response to irradiation by the electromagnetic radiation; extracting an SV-signal from the first image data; extracting a PPG-signal from the second image data; and determining a PTT based on the SV-signal and the PPG-signal.

In some embodiments, the method may further comprise determining a first time indicative of a systolic event at the first region based on the SV-signal; determining a second time indicative of an arrival of a cardiac pressure wave at the second region based on the PPG-signal; and determining the PTT based on a difference between the second time and the first time.

In some embodiments, the method may further comprise determining a physical distance between the first region and the second region; determining a PWV based on the PTT and the physical distance; and determining the physiological parameter based, at least in part, on the PWV or the PTT.

In a third aspect, a computer program product is provided. The computer program product contains a computer readable code configured such that, on execution by a suitable computer or processing unit, the computer or processing unit is caused to perform the method of the second aspect.

These and other aspects of the disclosure will be apparent from and elucidated with reference to the embodiment(s) described hereinafter.

BRIEF DESCRIPTION OF THE DRAWINGS

Same reference numerals refer to same elements or elements of similar function throughout the various figures. Furthermore, only reference numerals necessary for the description of the respective figure are shown in the figures. The shown embodiments represent only examples of how the invention can be carried out. This should not be construed as a limitation of the invention.

For a better understanding of the invention, and to show more clearly how it may be carried into effect, reference will now be made, by way of example only, to the accompanying drawings, in which:

Fig. 1 shows a schematic diagram of a system and a subject in accordance with an embodiment of the present invention;
Fig. 2 shows another schematic diagram of the system and the subject in accordance with an embodiment of the present invention;
Fig. 3A shows a schematic block diagram of a processor configured to extract an SV-signal in accordance with an embodiment of the present invention;
Fig. 3B shows a schematic block diagram of the processor configured to extract a PPG-signal in accordance with an embodiment of the present invention;
Fig. 4 shows a schematic block diagram of the processor configured to determine a PTT in accordance with an embodiment of the present invention;
Fig. 5 shows a graph illustrating an SV-signal and a PPG-signal in accordance with an embodiment of the present invention;
Fig. 6 shows a schematic block diagram of the processor configured to determine a pulse wave velocity in accordance with an embodiment of the present invention;
Fig. 7 shows a schematic block diagram of the processor configured to determine a physiological parameter in accordance with an embodiment of the present invention;
Figs. 8A and 8B show schematic diagrams of the system in accordance with another embodiment of the present invention;
Fig. 9 is schematic block diagram of an overview camera and the processor in accordance with another embodiment of the present invention;
Fig. 10 shows a schematic diagram of the system in accordance with another embodiment of the present invention; and
Fig. 11 is flowchart of a method for determining the physiological parameter of the subject in accordance with an embodiment of the present invention.

DETAILED DESCRIPTION OF THE EMBODIMENTS

As noted above, there is provided an improved system for determining the physiological parameter of the subject. In particular, there is provided a system for unobtrusively, reliably, and efficiently determining the physiological parameter of the subject which may allow an easy and practical application with minimum inconvenience to the subject as well as an increased accuracy and a method corresponding the same which addresses the existing problems.

As used herein, a "signal" comprises, but is not limited to, one or more electrical signals, optical signals, electromagnetic signals, analog and/or digital signals, one or more computer instructions, a bit and/or bit stream, or the like.

As used herein, a "physiological parameter" comprises, but is not limited to, a heart rate (HR), a respiration rate (RR), a blood pressure (BP), tachypneic respiratory dependent changes (BPchanges), a blood flow, a hemodynamic status, a vascular stiffness, a microvascular function, a hyper-/hypo-tension, an oxygen metabolism, a vascular tone, a vascular compliance, a venous status, a cardiac function, an atherosclerotic obstruction, a hydration status, a hemoglobin concentration, an oxygenation, and a blood viscosity.

As used herein, a "pulse transition time (PTT)" is a time required for a cardio-vascular pressure wave (CPW) to travel from a point closer to a heart of the subject to a point at a distal location (e.g., a finger or a head) from the heart.

As used herein, a "physical distance" is a length of an arterial segment between a point closer to a heart of a subject and a point at a distal location from the heart.

As used herein, a "systolic event" comprises any event which occurs during a systole of a cardiac cycle.

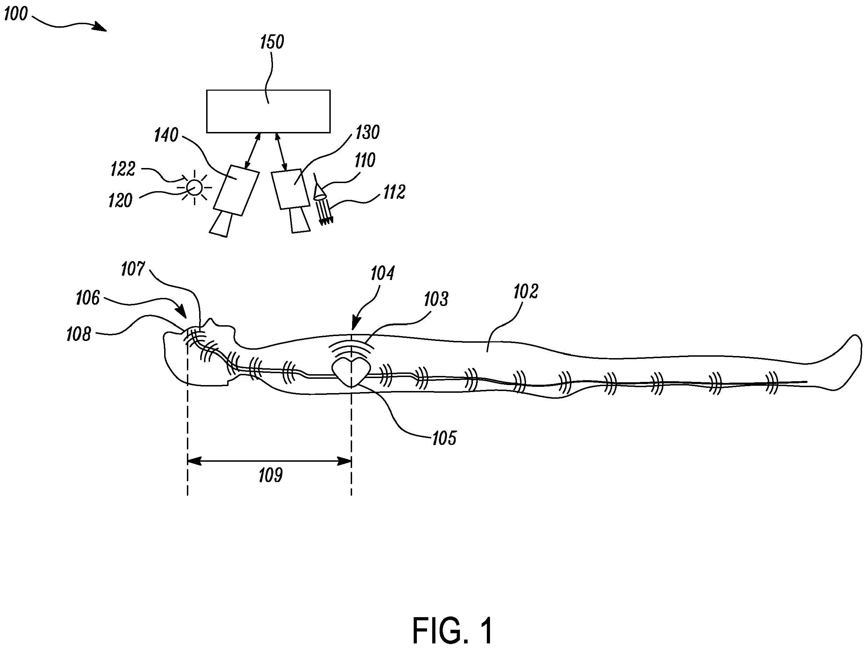

Fig. 1 shows a schematic view of a system 100 for determining a physiological parameter of a subject 102, according to an embodiment. In some examples, the physiological parameter, e.g., a BP, may serve as an indicator of a current health state of the subject 102. According to the invention, the subject 102 may be a living organism, such as a human or an animal. The subject 102 may be a patient.

In some embodiments, the system 100 may be located in a clinical environment. For example, the system 100 may be located in an operating room, an intensive care unit (ICU), a general ward, or any space/area within a healthcare facility, such as a hospital. In some embodiments, the system 100 may be located in a specific location within the healthcare facility where a continuous monitoring of the physiological parameter of the subject 102 is required. In some embodiments, the system 100 may be co-integrated in a hospital information system. In some other embodiments, the system 100 may be located in a kiosk, elderly care facility, a fitness center, or at a home of the subject 102.

The system 100 comprises a first radiation source 110, a second radiation source 120, a first detector 130, a second detector 140, and a processor 150. Each of the first radiation source 110, the second radiation source 120, the first detector 130, and the second detector 140 is located remotely from the subject 102. Therefore, the system 100 may be a contactless system for determining the physiological parameter of the subject 102. The system 100 may be suitable for the subject 102 with extreme skin sensitivity, such as a Neonatal Intensive Care Unit (NICU) patient with extremely fragile skin, a premature baby, or a patient with extensive burns, who may require constant monitoring of the physiological parameter.

Fig. 1 further illustrates a first region 104 proximal to a heart 105 of the subject 102 and a second region 106 of the subject 102 remote from the first region 104 of the subject 102.

In some examples, a torso of the subject 102 may comprise the first region 104. For example, the first region 104 may comprise a chest or a back of the subject 102. In some cases, the first region 104 may be in left or right sides of the subject 102. In some examples, a portion of a head of the subject 102 may comprise the second region 106. For example, the second region 106 may comprise a forehead or a cheek of the subject 102. In some other examples, a portion of a limb of the subject 102 may comprise the second region 106. For example, the limb of the subject 102 may comprise a hand, an arm, a wrist, or a foot of the subject 102.

Fig. 1 further illustrates a physical distance 109 between the first region 104 and the second region 106. The physical distance 109 may correspond to a length of an arterial segment between the first region 104 and the second region 106. In some embodiments, the physical distance 109 may be determined by measuring a distance between the centers of the first region 104 and the second region 106. In the illustrated example of Fig. 1, the physical distance 109 is between the chest and the head of the subject 102. In some cases, the physical distance 109 may be predetermined based on previous measurements or examinations of the subject 102. In some embodiments, the system 100 determines the physical distance 109 between the first region 104 and the second region 106. In some other embodiments, the processor 150 of the system 100 may determine the physical distance 109.

Fig. 1 further illustrates mechanical waves 103 and cardiovascular pressure waves (CPW) 107. The CPW 107 originate from the heart 105 and generally travel through arteries. Generally, the CPW 107 result in variations in blood volume in the arteries of the subject 102. The mechanical waves 103 originate and are induced from the heart 105 at the first region 104. The mechanical waves 103 may travel through the tissues towards a surface (e.g., a chest) of the subject 102. Generally, the mechanical waves 103 result in micro-vibrations on the surface of the subject 102. The mechanical waves 103 may further travel through the tissues and a skeleton of the subject 102. In some cases, mechanical waves may also originate from the CPW 107 at regions remote from the first region 104 (e.g., the second region 106). The mechanical waves 103 may comprise various types of waves, such as acoustic waves, and/or Primarywaves (P-waves), Secondary-waves (S-waves), and so forth.

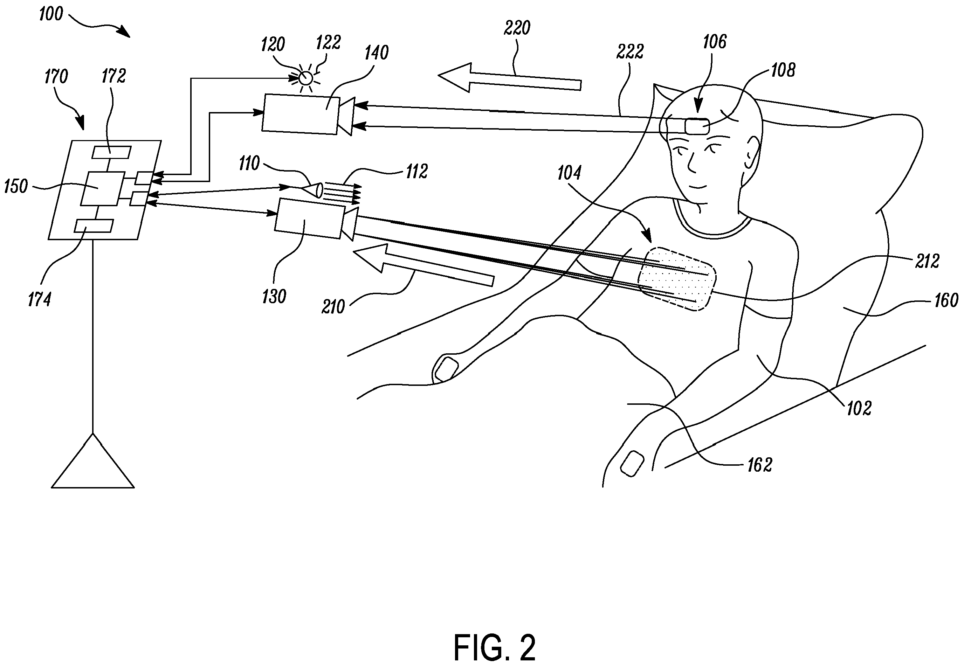

Fig. 2 illustrates a schematic view of the system 100, according to another embodiment. In the illustrated example shown in Fig. 2, the subject 102 is the patient lying on a bed 160, for example in the healthcare facility. In some other examples, the subject 102 may be a neonate or a premature infant lying in an incubator.

Referring to Figs. 1 and 2, the first radiation source 110 is configured to emit a laser radiation 112 towards the first region 104 of the subject 102. In some embodiments, the first radiation source 110 may be manually adjusted to emit the laser radiation 112 towards the first region 104 of the subject 102. In some other embodiments, the first radiation source 110 may be automatically adjusted (e.g., by the processor 150) to emit the laser radiation 112 towards the first region 104 of the subject 102. The first region 104 may be generally covered by textiles, e.g., a blanket 162 and/or a clothing of the subject 102. Therefore, a skin at the first region 104 may not be exposed.

In some embodiments, the first radiation source 110 is a coherent light source. In some embodiments, the first radiation source 110 is a laser, such as a diode laser. In some embodiments, the first radiation source 110 is a vertical-cavity surface-emitting laser (VCSEL). In some examples, the first radiation source 110 may be detachably attached to the first detector 130. In some embodiments, the first radiation source 110 may disposed in any suitable location remote from the subject 102 and configured to emit the laser radiation 112 towards the first region 104 of the subject 102.

The second radiation source 120 is configured to emit an electromagnetic radiation 122 towards the second region 106 of the subject 102. In some embodiments, the second radiation source 120 may be manually adjusted to emit the electromagnetic radiation 122 towards the second region 106 of the subject 102. In some other embodiments, the second radiation source 120 may be automatically adjusted (e.g., by the processor 150) to emit the electromagnetic radiation 122 towards the second region 106 of the subject 102.

The second region 106 may not be usually not covered by the blanket 162 and/or the clothing of the subject 102. Therefore, a skin 108 at the second region 106 may be exposed to the electromagnetic radiation 122. The skin 108 at the second region 106 reflects or transmit at least a portion of the electromagnetic radiation 122.

In some embodiments, the second radiation source 120 comprises a lamp, a light-emitting diode (LED) light source, or an organic light emitting diode (OLED). In some embodiments, the second radiation source 120 comprises an infrared light source. In some embodiments, the second radiation source 120 comprises a visible light source. In some embodiments, the second radiation source 120 may comprise any light source that emits light in a wavelength range from about 400 nanometer (nm) to 1000 nm. In some embodiments, the second radiation source 120 may comprise a laser, such as a diode laser or a VCSEL. In some embodiments, the second radiation source 120 may be a dedicated light source disposed on the second detector 140. In some examples, the second radiation source 120 may be detachably attached to the second detector 140. In some embodiments, the second radiation source 120 may be any ambient light source configured to emit the electromagnetic radiation 122 towards the second region 106 of the subject 102.

The first detector 130 is configured to acquire a first image data 210 from a laser speckle pattern 212 generated at the first region 104 in response to irradiation by the laser radiation 112 from the first radiation source 110.

In some embodiments, the first detector 130 is a camera. In some embodiments, the first detector 130 is an RGB video camera. In some embodiments, the camera may comprise a photo sensor-array, such as a complementary metal oxide semiconductor (CMOS) image-sensor or a charge-coupled device (CCD) image-sensor. In some embodiments, the first detector 130 comprises a defocused lens.

In some embodiments, the system 100 is configured to adjust a focus of the camera to acquire the first image data 210 from the laser speckle pattern 212. In some embodiments, the system 100 may be configured to defocus the camera in order to acquire the first image data 210 from the laser speckle pattern 212. In some embodiments, the system 100 may adjust the focus of the camera based on the requirements of the system 100, such as a field of view of the first detector 130, minimal resolution and signal-to-noise requirements, as well as a distance of the first detector 130 from the first region 104.

In some embodiments, the first image data 210 may be a video data acquired from the laser speckle pattern 212 by the first detector 130. In some embodiments, the first detector 130 may be configured to record the first image data 210 acquired from the laser speckle pattern 212.

The first image data 210 comprises a pattern referred to as the laser speckle pattern 212. When the first region 104 is illuminated by the laser radiation 112, minute path length differences in the reflected light from the first region 104 may result in the laser speckle pattern 212 that may be observed by the first detector 130. The first image data 210 acquired by a focused lens of the camera may include small dots. The first image data 210 acquired by the defocused lens of the camera may include relatively large (typically circular) areas with distinct and sharp laser speckle pattern 212. The laser speckle pattern 212 fluctuates or varies at a rate dependent on a degree of micro-vibrations in the first region 104.

As discussed above, the mechanical waves 103 may cause the micro-vibrations in the first region 104. When measured over time, the variations in the laser speckle pattern 212 resulting from the mechanical waves 103 may reveal a heartbeat or a cardiac cycle of the subject 102. A variation in the laser speckle pattern 212 may be large even due to a micro-vibration at the first region 104. In other words, even a small change in a profile or a contour of the first region 104 irradiated by the laser radiation 112 may give rise to a large variation in the laser speckle pattern 212. The first detector 130 is configured to detect such micro-vibrations at the first region 104 and acquire the first image data 210 from the laser speckle pattern 212 generated at the first region 104.

Further, the first detector 130 may detect the micro-vibrations at the first region 104 and acquire the first image data 210 from the laser speckle pattern 212 generated at the first region 104 even when the first region 104 is covered (e.g., with the blanket 162) since the micro-vibrations are carried from the skin at the first region 104 to the cover. Therefore, the first detector 130 may detect the micro-vibrations resulting from the mechanical waves 103 at the first region 104. However, the first detector 130 may not be able to accurately detect the micro-vibrations resulting from the mechanical waves 103 originating from the heart 105 at regions remote from the first region 104 (e.g., the second region 106) as the micro-vibrations at such regions may also be due to the CPW 107 and the mechanical waves transmitted by the body or the skeleton of the subject 102.

The first radiation source 110 and the first detector 130 may therefore enable a distant and contactless measurement of the micro-vibrations induced by the mechanical waves 103 at the first region 104. Further, the first radiation source 110 and the first detector 130 may enable a measurement of the micro-vibrations induced by the mechanical waves 103 even when the skin at the first region 104 is not exposed, i.e., covered.

The second detector 140 is configured to acquire a second image data 220 from a subject radiation 222 reflected from or transmitted through the skin 108 at the second region 106 in response to irradiation by the electromagnetic radiation 122 from the second radiation source 120.

In some embodiments, the second detector 140 is a camera. In some embodiments, the camera is an RGB video camera. In some embodiments, the second detector 140 is a photodetector. In some embodiments, the camera may comprise a photo sensor-array, such as a CMOS image-sensor or a CCD image-sensor, which may also operate in a specific spectral range (e.g., a visible range or a near infrared range) or provide the second image data 220 for different spectral ranges. The camera may provide an analog or a digital signal.

In some embodiments, the second image data 220 may be a video data acquired by the second detector 140. In some embodiments, the second detector 140 may be configured to record the second image data 220 acquired from the subject radiation 222.

The second image data 220 may comprise a plurality of image pixels having associated pixel values. Particularly, the second image data 220 may comprise pixels representing light intensity values captured with different photosensitive elements of a photo sensor. These photosensitive elements may be sensitive in a specific spectral range (i.e., representing a specific color). The second image data 220 may comprise at least some image pixels representative of the second region 106. Thereby, an image pixel may correspond to one photosensitive element of a photo-detector and its (analog or digital) output, or may be determined based on a combination (e.g., through binning) of a plurality of the photosensitive elements.

The second detector 140 may evaluate a time-variant change of light reflectance or transmission at the second region 106 based on the principle that blood absorbs the electromagnetic radiation 122 more than surrounding tissue, such that variations in blood volume with every heartbeat may affect the transmission or the reflectance of the subject radiation 222 correspondingly. When measured over time, the variations in the subject radiation 222 resulting from the variations in blood volume may reveal the heartbeat or the cardiac cycle of the subject 102. The variations in the subject radiation 222 at the second region 106 are caused by the CPW 107. The second detector 140 is configured to detect such variations in the blood volume at the skin 108 at the second region 106 and acquire the second image data 220 from the subject radiation 222 emitted from the second region 106. Since the second detector 140 is configured to acquire the second image data 220 from the subject radiation 222 reflected from or transmitted through the skin 108 of the subject 102, the skin 108 at the second region 106 may be required to be exposed. As discussed above, the second region 106 may comprise regions remote from the first region 104 which are usually not covered by blanket or clothing. Therefore, the second detector 140 may detect the variations in the blood volume at the skin 108 at the second region 106. Further, the second detector 140 may not be able to measure the variations in the blood volume at the first region 104. Specifically, the second detector 140 may not be able to measure the variations in the blood volume at the first region 104 as the skin at the first region 104 is not typically exposed.

The processor 150 is communicably coupled to the first detector 130 and the second detector 140. The processor 150 processes the first image data 210 and the second image data 220. In some embodiments, the processor 150 may be communicably coupled to the first radiation source 110 and the second radiation source 120.

In some embodiments, the processor 150 can be implemented as a single entity, such as a microcontroller or a field programmable gate array (FPGA), or may also be implemented as a distributed processing device comprising a plurality of separate processing entities or even a cloud-based solution. The processor 150 may also be shared with other applications.

In some embodiments, the system 100 further comprises an interface 170. In some embodiments, the interface 170 comprises the processor 150. In some embodiments, the interface 170 is configured to receive the first image data 210 and the second image data 220 from the first and second detectors 130, 140, respectively. In some embodiments, the interface 170 may be configured to receive the first image data 210 and the second image data 220 from the first and second detectors 130, 140, respectively, via a wired and/or wireless network. The network may transmit computer program instructions, data structures, program modules or other data over a wired or wireless substance by propagating a modulated data signal, such as a carrier wave or other transport mechanism, over the wired or wireless substance. The term "modulated data signal", as used herein, means a signal that has one or more of its characteristics set or changed in such a manner as to encode information in the signal, thereby changing the configuration or state of the receiving device of the signal. In some embodiments, the network may comprise a radio frequency network, or a cellular network, such as global system for mobile communications (GSM), general packet radio service (GPRS), 3G, evolution-Data Optimized (EVDO), long-Term Evolution (LTE), 4G, 5G, mesh, or any other network. In some embodiments, the network may comprise a Bluetooth®, Near Field Communication (NFC) network, mesh network, infrared, ultraband, intranet, internet, and so forth.

In some embodiments, the interface 170 may comprise displays, buttons, touchscreens, keyboards and/or other human machine interface (HMI) means. In some embodiments, the interface 170 may enable a user, for example, a doctor, a nurse, or any other medical professional to provide a user input in order to change settings of the first radiation source 110, the second radiation source 120, the first detector 130, and/or the second detector 140 of the system 100.

In some embodiments, the first radiation source 110 may be manually adjusted based on the user input. In some embodiments, the second radiation source 120 may be manually adjusted based on the user input. In some embodiments, the physical distance 109 may be manually provided as the user input in cases the physical distance 109 is known by the user and/or the subject 102.

In some embodiments, the system 100 may comprise a memory 172. In the illustrated embodiment of Fig. 2, the interface 170 comprises the memory 172. In some embodiments, the memory 172 is communicably coupled to the processor 150.

In some embodiments, the system 100 may further comprise a display 174. In the illustrated embodiment of Fig. 2, the interface 170 comprises the display 174. In some examples, the display 174 may be configured to display the physiological parameter.

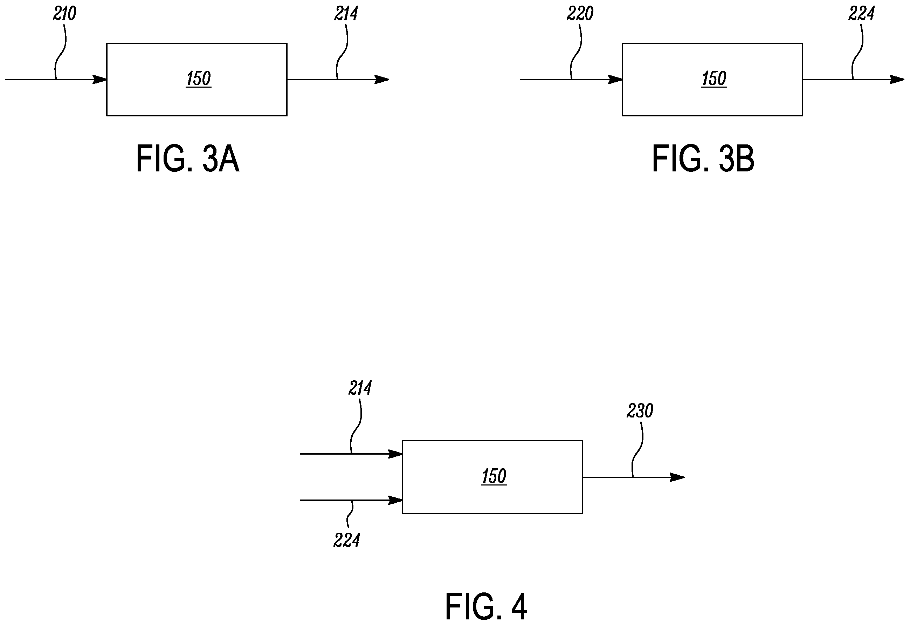

Referring to Fig. 3A, the processor 150 is configured to extract an SV-signal 214 from the first image data 210. The SV-signal 214 may correspond to a signal representing the variations in the laser speckle pattern 212 resulting from the mechanical waves 103 (shown in Fig. 1). In other words, the SV-signal 214 may correspond to a signal representing the micro-vibrations from the mechanical waves 103. The SV-signal 214 may be determined based on a time series of the first image data 210. In some embodiments, the display 174 (shown in Fig. 2) may be configured to display the SV-signal 214.

Referring to Fig. 3B, the processor 150 is configured to extract a PPG-signal 224 from the second image data 220. The PPG-signal 224 may correspond to a signal representing variations in the light intensity of the subject radiation 222 resulting from the CPW 107 (shown in Fig. 1). In other words, the PPG-signal 224 may correspond to a signal representing the variations in the blood volume resulting from the CPW 107. The PPG-signal 224 may be determined based on a time series of the second image data 220. In some embodiments, the display 174 (shown in Fig. 2) may be configured to display the PPG-signal 224.

Referring to Fig. 4, the processor 150 is configured to determine a PTT 230 based on the SV-signal 214 and the PPG-signal 224. In some embodiments, the display 174 (shown in Fig. 2) may be configured to display the PTT 230. The PTT 230 based on the SV-signal 214 may be more accurate than a pulse travel time based on an ECG-signal comprising a pre-ejection period (PEP). The PEP in the ECG-signal may lead to a distortion of the pulse travel time as the PEP typically has a variable duration. Further, the PPG-signal 230 may not be susceptible to the mechanical waves transmitted through the body or the skeleton of the subject 102 (shown in Fig. 1).

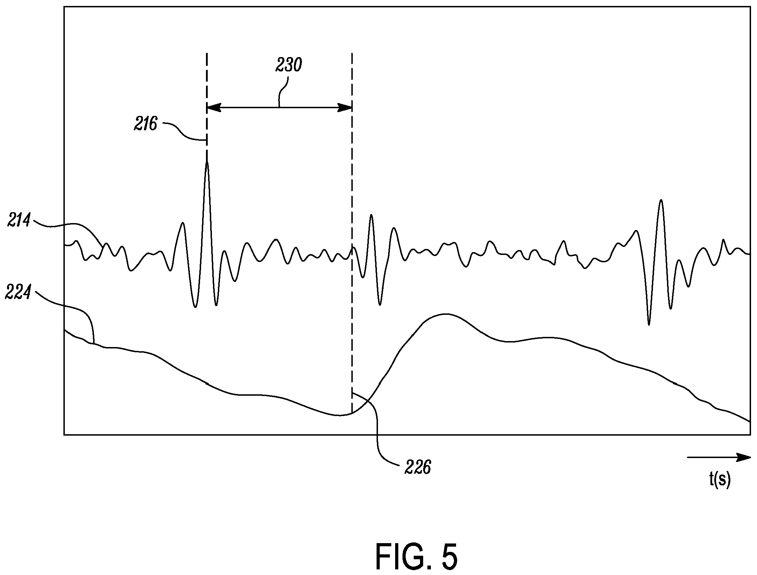

Fig. 5 illustrates the SV-signal 214 and the PPG-signal 224, according to an embodiment. Referring to Figs. 1 and 5, in some embodiments, the processor 150 is further configured to determine a first time 216 indicative of a systolic event at the first region 104 based on the SV-signal 214. In some embodiments, the systolic event comprises an onset of a systolic phase. In some embodiments, the systolic event comprises an aortic valve opening. In some embodiments, the first time 216 may be indicative of a cardiac ejection of blood or a start of the CPW 107 at the first region 104. Therefore, the first time 216 may be based on the mechanical waves 103 at the first region 104.

In some embodiments, the processor 150 is further configured to determine a second time 226 indicative of an arrival of the CPW 107 at the second region 106 based on the PPG-signal 224. The arrival of the CPW 107 at the second region may be indicated by a systolic onset at the second region 106. Therefore, the second time 226 may be based on the CPW 107 at the second region 106.

In some embodiments, the processor 150 is further configured to determine the PTT 230 based on a difference between the second time 226 and the first time 216. Generally, for determining a pulse travel time, a time of arrival of the CPW 107 at the first region and a time of arrival of the CPW 107 at the second region 106 are used. For accurate measurement of the pulse travel time, precise time of arrival of the CPW 107 at the first region 104 and precise the time of arrival of the CPW 107 at the second region 106 are required. However, the CPW 107 cannot be measured at the heart 105. The measurement of the time of arrival of the CPW 107 at two distant remote regions from the heart 105 may comprise time delays. The mechanical waves 103, however, may be used to measure the systolic event at the first region 104 without any delay. Therefore, the PTT 230 based on the difference between the second time 226 and the first time 216 may be accurate and reliable.

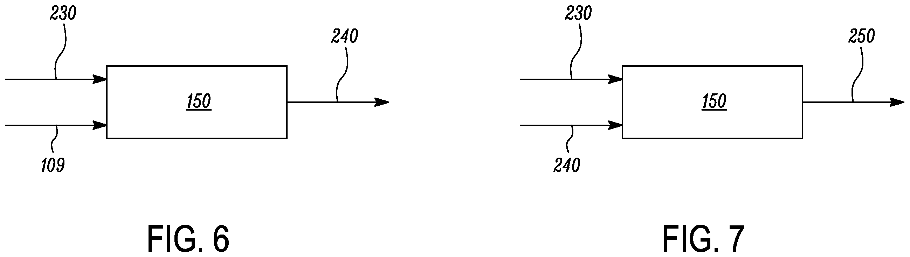

Referring to Fig. 6, the processor 150 is configured to determine a PWV 240 based on the PTT 230 and the physical distance 109. Specifically, the PWV 240 may be determined in the following way:

$$PWV=D/PTT,$$

where D is the physical distance 109.

Referring to Fig. 7, the processor 150 is further configured to determine a physiological parameter 250 based, at least in part, on the PWV 240 or the PTT 230. The PTT 230 may be a valuable parameter for determining the physiological parameter 250. In some cases, the physiological parameter 250 may provide assessment of the cardiovascular system of the subject 102 (shown in Fig. 1). In some other cases, the physiological parameter 250 may be used for respiratory sleep monitoring of the subject 102. In some embodiments, the display 174 (shown in Fig. 2) may be configured to display the physiological parameter 250.

In some cases, determination of the physiological parameter 250 based on the PWV 240 may be more accurate compared to the determination of the physiological parameter 250 based on the PTT 230, as described in the paper of Mishra, Biswajit, and Nilesh Thakkar, "Cuffless blood pressure monitoring using PTT and PWV methods" 2017 International Conference on Recent Innovations in Signal processing and Embedded Systems (RISE). IEEE, 2017.

In some cases, the physiological parameter 250 may be the BP. The BP may be an indispensable physiological parameter 250 to evaluate health of the subject 102. The BP of the subject 102 may be determined from either the PTT 230 or the PWV 240. The PTT 230 may be inversely proportional to the BP. As the artery stiffens with the BP, the travel velocity of the CPW 107 increases and the PTT 230 decreases. Therefore, a non-invasive, an unobtrusive, a contactless, and a continuous monitoring of the BP is possible based on the PTT 230.

In some embodiments, unloading of respiratory muscles induced by non-invasive ventilation (NIV) and the increase in respiratory effort may also be accurately determined based on the PTT 230. The PTT 230 may further be useful to differentiate between obstructive respiratory events occurring under NIV during sleep. In some embodiments, the PTT 230 is also used to determine inspiratory effort of the subject 102 with obstructive sleep apnea.

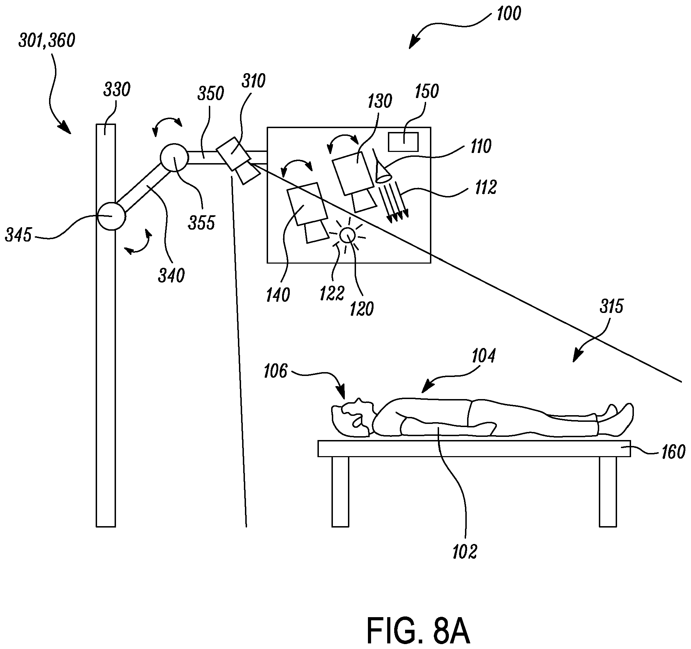

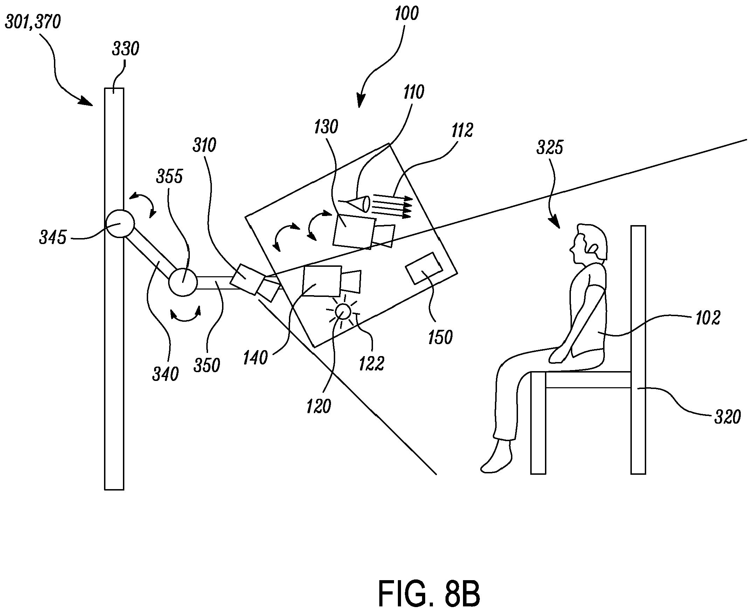

Figs. 8A and 8B illustrate schematic views of the system 100 for determining the physiological parameter 250 (shown in Fig. 7) of the subject 102, according to another embodiment. In some embodiments, the system 100 may be located in the kiosk.

In the illustrated embodiments of Figs. 8A and 8B, the system 100 further comprises an overview camera 310 communicably coupled to the system 100. Fig. 8A illustrates the subject 102 in a first position 315. In the illustrated example of Fig. 8A, the first position 315 is a supine position. Fig. 8B illustrates the subject 102 in a second position 325. In the illustrated example of Fig. 8B, the second position 325 is a sitting position. In some examples, the subject 102 may be seated on a chair 320 during a dialysis procedure. In some other embodiments, the first and second positions 315, 325 of the subject 102 may further comprise a prone position, a standing position, a left lateral recumbent position, or a right lateral recumbent position.

In some embodiments, the system 100 further comprises a robotic device 301 In some embodiments, each of the first radiation source 110, the second radiation source 120, the first detector 130, and the second detector 140 may be located or mounted on the robotic device 301. In some embodiments, the overview camera 310 may also be located on the robotic device 301. In some other embodiments, the overview camera 310 may be fixed to a ceiling, or a wall of an environment of the system 100.

In some embodiments, the robotic device 301 may be a mobile robotic device. In some embodiments, the mobile robotic device may comprise a wheeled robot, a legged robot, an unmanned aerial vehicle, and so forth. In some embodiments, the robotic device 301 may be a fixed robotic device. In some embodiments, the fixed robotic device may be fixed to a ceiling, a wall, and/or a floor of the environment of the system 100.

In some embodiments, the robotic device 301 may comprise a vertical structure 330. The robotic device 301 further comprises a first robotic arm 340 slidably and rotatably attached to the vertical structure 330 via a first joint 345. In some embodiments, the robotic device 301 further comprises a second robotic arm 350 rotatably attached to the first robotic arm 340 via a second joint 355. In some embodiments, the robotic device 301 may be communicably coupled to the processor 150 of the system 100.

In the illustrated embodiment of Figs. 8A and 8B, each of the first radiation source 110, the second radiation source 120, the first detector 130, and the second detector 140 are located on the second robotic arm 350. In some embodiments, the first radiation source 110, the second radiation source 120, the first detector 130, and the second detector 140 are movably attached to the second robotic arm 350. In some other embodiments, the first radiation source 110, the second radiation source 120, the first detector 130, and the second detector 140 are fixedly attached to the second robotic arm 350.

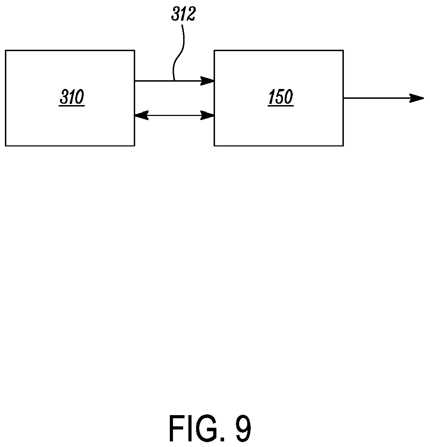

Referring to Figs. 8A, 8B and 9, the overview camera 310 is communicably coupled to the processor 150. In some other embodiments, the overview camera 310 may be communicably coupled to any other component of the system 100, such as a controller (not shown) or the interface 170 (shown in Fig. 2). In some cases, the controller may be disposed in the interface 170. In some cases, the controller may comprise the processor 150. In some other cases, the controller may be a separate component comprising one or more microprocessors, a memory, and other suitable components for performing various processing and controlling functions of the system 100. The overview camera 310 is configured to generate an image 312 of the subject 102. In some embodiments, the physical distance 109 (shown in Fig. 1) may be determined by the overview camera 310. Specifically, the physical distance 109 may be determined by measuring the distance between the centers of the first region 104 and the second region 106 based on the image 312. If the overview camera 310 is calibrated, in some embodiments, the physical distance 109 may be determined by a pixel distance between the center of the first region 104 and the center of the second region 106. If the overview camera 310 is not calibrated, in some embodiments, the physical distance 109 may be determined based a pixel distance between the center of the first region 104 and the center of the second region 106, and an object with known dimensions in the image 312 at a known distance from the overview camera 310. In some embodiments, the physical distance 109 between the first region 104 and the second region 106 may be determined using the pinhole projection formula.

In some embodiments, the system 100 is further configured to adjust a position of the first detector 130 and/or a position of the second detector 140 based on the image 312 of the subject 102. Specifically, in some embodiments, the system 100 is further configured to determine a position (e.g., the first position 315 or the second position 325) of the subject 102 based on the image 312 of the subject 102. In some embodiments, the system 100 is configured to adjust the position of the first detector 130 and/or the position of the second detector 140 based, at least in part, on the position (e.g., the first position 315 or the second position 325) of the subject 102. In some embodiments, the processor 150 of the system 100 may be configured to adjust the position of the first detector 130 and/or the position of the second detector 140 based on the image 312 of the subject 102. However, in some other embodiments, any other component, such as the controller or the interface 170 (shown in Fig. 2) of the system 100, may be configured to adjust the position of the first detector 130 and/or the position of the second detector 140 based on the image 312 of the subject 102.

In some embodiments, the system 100 may further adjust a position of the robotic device 301 in order to adjust the position of the first detector 130 and/or the position of the second detector 140. For example, the robotic device 301 may be in a first position 360 (shown in Fig. 8A) when the subject 102 in the first position 315 and the robotic device 301 may be in a second position 370 (shown in Fig. 8B) when the subject 102 in the second position 325. In some embodiments, the system 100 may adjust a position of the first robotic arm 340 and a position of the second robotic arm 350 in order to adjust the position of the first detector 130 and/or the position of the second detector 140. In some embodiments, the first robotic arm 340 and the second robotic arm 350 may be mechanically, pneumatically and/or hydraulically actuated by the system 100. In some embodiments, the first robotic arm 340 and the second robotic arm 350 may be actuated by the processor 150 of the system 100. In some other embodiments, the first robotic arm 340 and the second robotic arm 350 may be actuated by any other component, such as control circuitry of the robotic device 310 or the interface 170 of the system 100. The system 100 may transmit control signals to one or more actuators (not shown) to control each of the first and second robotic arms 340, 350.

In some embodiments, the system 100 is further configured to adjust a position of the first radiation source 110 and/or a position of the second radiation source 120 based on the image 312 of the subject 102. In some embodiments, the processor 150 of the system 100 is configured to adjust the position of the first radiation source 110 and/or the position of the second radiation source 120 based on the image 312 of the subject 102. In some other embodiments, any other component, such as the controller or the interface 170 (shown in Fig. 2) of the system 100, is configured to adjust the position of the first radiation source 110 and/or the position of the second radiation source 120 based on the image 312 of the subject 102. For example, the processor 150 may be further configured to adjust the position of the first radiation source 110 and/or the position of the second radiation source 120 based, at least in part, on the position (e.g., the first position 315 or the second position 325) of the subject 102 determined based on the image 312. In some embodiments, the system 100 (e.g., via the processor 150) may adjust the position of the first robotic arm 340 and the position of the second robotic arm 350 in order to adjust the position of the first radiation source 110 and/or the position of the second radiation source 120.

In some embodiments, the system 100 is further configured to determine a location of the first region 104 and a location of the second region 106 based on the image 312 of the subject 102. For example, the location of the first region 104 and the location of the second region 106 may depend upon a height of the subject 102. In another example, the location of the first region 104 and the location of the second region 106 may also depend upon a size of the bed 160, or a height of the chair 320. In some embodiments, the system 100 may be configured to determine the location of the first region 104 and the location of the second region 106 using an artificial intelligence (AI) processing of the image 312. For example, various anatomical elements, such as the head, the chest, and the hand of the subject 102 may be distinguished and determined based on the AI processing. In some embodiments, the AI processing may be performed by the processor 150.

In some embodiments, the system 100 is further configured to adjust the position of the first detector 130 based on the location of the first region 104. In some embodiments, the system 100 is further configured to adjust the position of the second detector 140 based on the location of the second region 106.

Therefore, adjustment of the position of the first detector 130 and/or the position of the second detector 140 may be automatically done based on the position of the subject 102 without intervention of the user.

In some embodiments, the overview camera 310 may be movably mounted on the second robotic arm 350. The system 100 may adjust a position of the overview camera 310 until a suitable image of the subject 102 is captured. In some embodiments, the interface 170 (shown in Fig. 2) may be configured to adjust the position of the overview camera 310 until a suitable image of the subject 102 is captured based on the user input.

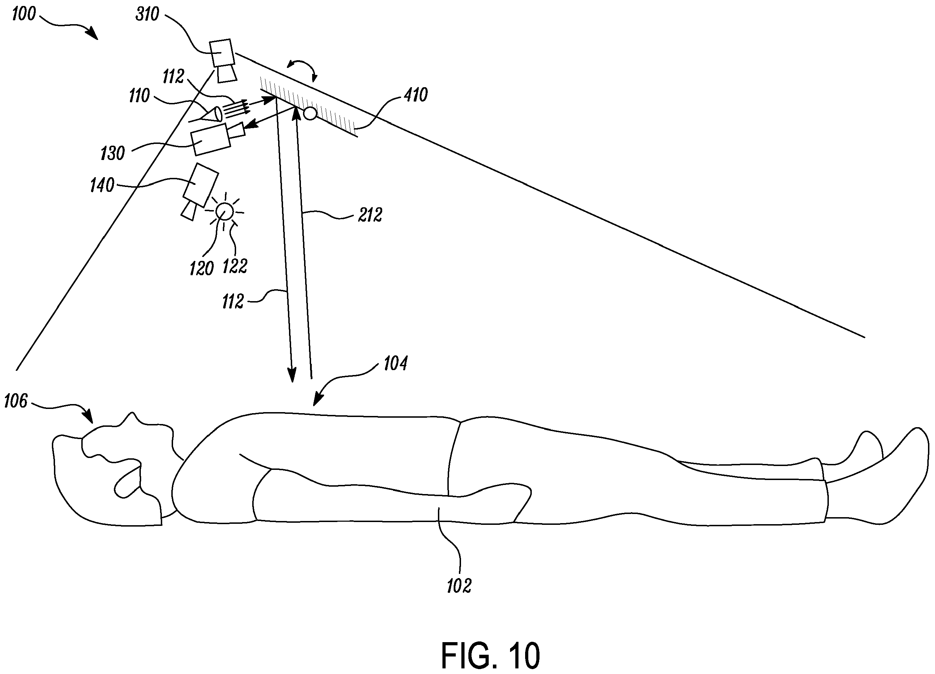

Fig. 10 illustrates a schematic view of the system 100 for determining the physiological parameter 250 (shown in Fig. 7) of the subject 102, according to another embodiment.

In the illustrated embodiment of Fig. 10, the system 100 further comprises at least one movable mirror 410. In some embodiments, the at least one movable mirror 410 may be required when the size of the bed 160 (shown in Fig. 8A) is very large for a single subject 102. In some embodiments, the system 100 may comprise the robotic device 301 as shown in Figs. 8A and 8B in addition to the at least one movable mirror 410.

In the illustrated embodiment of Fig. 10, the at least one movable mirror 410 is configured to guide the laser radiation 112 from the first radiation source 110 to the subject 102. Specifically, the at least one movable mirror 410 is configured to guide the laser radiation 112 from the first radiation source 110 to the first region 104 of the subject 102. In some embodiments, the system 100 is further configured to adjust a position of the at least one movable mirror 410 based on the image 312 (shown in Fig. 9) of the subject 102. Specifically, the system 100 is configured to adjust the position of the at least one movable mirror 410 based on the location of the first region 104 of the subject 102. In some embodiments, the processor 150 (shown in Fig. 9) or the interface 170 (shown in Fig. 2) of the system 100 may be configured to adjust the position of the at least one movable mirror 410 based on the location of the first region 104 of the subject 102. In some other embodiments, any other component (e.g., the controller) of the system 100 may be configured to adjust the position of the at least one movable mirror 410 based on the location of the first region 104 of the subject 102.

In some embodiments, the at least one movable mirror 410 is configured to guide the electromagnetic radiation 122 from the second radiation source 120 to the subject 102. Specifically, the at least one movable mirror 410 is configured to guide the electromagnetic radiation 122 from the second radiation source 120 to the second region 106 of the subject 102. In some embodiments, the system 100 is further configured to adjust the position of the at least one movable mirror 410 based on the location of the second region 104 of the subject 102. In some embodiments, the processor 150 (shown in Fig. 9) or the interface 170 (shown in Fig. 2) of the system 100 may be configured to adjust the position of the at least one movable mirror 410 based on the location of the second region 104 of the subject 102. In some other embodiments, any other component (e.g., the controller) of the system 100 may be configured to adjust the position of the at least one movable mirror 410 based on the location of the second region 104 of the subject 102.

In some embodiments, the at least one movable mirror 410 is configured to guide each of the laser radiation 112 from the first radiation source 110 to the first region 104 of the subject 102 and the electromagnetic radiation 122 from the second radiation source 120 to the second region 106 of the subject 102. In these embodiments, the at least one movable mirror 410 may comprise one or more movable mirrors, for example, two movable mirrors.

In some embodiments, the at least one movable mirror 410 is further configured to guide a radiation from the subject 102 to the first detector 130 and/or the second detector 140. Specifically, the at least one movable mirror 410 is configured to guide a radiation corresponding to the laser speckle pattern 212 from the subject 102 to the first detector 130 and/or the subject radiation 222 from the subject 102 to the second detector 140. More specifically, the at least one movable mirror 410 is configured to guide the radiation corresponding to the laser speckle pattern 212 from the first region 104 of the subject 102 to the first detector 130 and/or the subject radiation 222 from the second region 106 of the subject 102 to the second detector 140.

In the illustrated embodiment of Fig. 10, the at least one movable mirror 410 is configured to guide the radiation corresponding to the laser speckle pattern 212 from the subject 102 to the first detector 130.

Therefore, in some embodiments, the system 100 may comprise at least one, at least two, at least three, or at least four movable mirrors. The system 100 is further configured to adjust the position of each movable mirror 410 based on the image 312 (shown in Fig. 9) of the subject 102. Specifically, the system 100 is further configured to adjust the positions of the respective movable mirrors 410 based on the location of the first region 104 and the location of the second region 106. In some embodiments, the processor 150 or the interface 170 of the system 100 may be configured to adjust the position of each movable mirror 410 based on the image 312. In some other embodiments, any other component (e.g., the controller) of the system 100 may be configured to adjust the position of each movable mirror 410 based on the image 312.

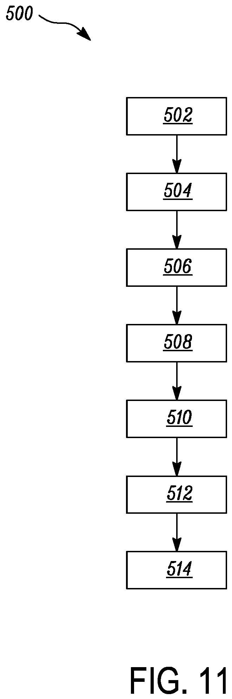

Fig. 11 shows a flowchart depicting a method 500 for determining the physiological parameter 250 (shown in Fig. 7) of the subject 102 (shown in Fig. 1).

In some embodiments, the system 100 (shown in Fig. 1) may comprise one or more of software and/or hardware modules that are each configured to perform, or are for performing, individual or multiple steps of the method 500 described herein. In some embodiments, the memory 172 (shown in Fig. 2) may be configured to store program code that can be executed by the system 100 to perform the various steps described herein. In some embodiments, the processor 150 may be configured to perform the various steps described herein. In some other embodiments, the controller may be configured to perform the various steps described herein. In some other cases, some of the steps may be performed by the processor 150, while the other steps may be performed by the controller. The method 500 will be described with reference to Figs. 1 to 10.

The method 500 comprises, at step 502, emitting the laser radiation 112 towards the first region 104 of the subject 102 proximal to the heart 105 of the subject 102.

The method 500 comprises, at step 504, emitting the electromagnetic radiation 122 towards the second region 106 of the subject 102 remote from the first region 104 of the subject 102.

The method 500 comprises, at step 506, acquiring the first image data 210 from the laser speckle pattern 212 generated at the first region 104 in response to irradiation by the laser radiation 112.

The method 500 comprises, at step 508, acquiring the second image data 220 from the subject radiation 222 reflected from or transmitted through the skin 108 at the second region 106 in response to irradiation by the electromagnetic radiation 122.

The method 500 comprises, at step 510, extracting the SV-signal 214 from the first image data 210.

The method 500 comprises, at step 512, extracting the PPG-signal 224 from the second image data 220.

The method 500 comprises, at step 514, determining the PTT 230 based on the SV-signal 214 and the PPG-signal 224.

In some embodiments, the method 500 further comprises determining the first time 216 indicative of the systolic event at the first region 104 based on the SV-signal 214. In some embodiments, the method 500 further comprises determining the second time 226 indicative of the arrival of the CPW at the second region 106 based on the PPG-signal 224. In some embodiments, the method 500 comprises determining the PTT 230 based on the difference between the second time 226 and the first time 216. In some embodiments, the method 500 further comprises determining the physical distance 109 between the first region 104 and the second region 106. In some embodiments, the method 500 further comprises determining the PWV 240 based on the PTT 230 and the physical distance 109. In some embodiments, the method 500 comprises determining the physiological parameter 250 based, at least in part, on the PWV 240

or the PTT 230.

In some embodiments, the method 500 further comprises adjusting the focus of the first detector 130 to acquire the first image data 210 from the laser speckle pattern 212.

In some embodiments, the method 500 further comprises generating, via the overview camera 310, the image 312 of the subject 102 and adjusting the position of the first detector 130 and/or the position of the second detector 140 based on the image 312 of the subject 102.

In some embodiments, the method 500 further comprises determining the position (e.g., the first position 315 or the second position 325) of the subject 102 based on the image 312 of the subject 102, and adjusting the position of the first detector 130 and/or the position of the second detector 140 based, at least in part, on the position of the subject 102.

In some embodiments, the method 500 further comprises determining the location of the first region 104 and the location of the second region 106 based on the image 312 of the subject 102. In some embodiments, the method 500 further comprises adjusting the position of the first detector 130 based on the location of the first region 104 and adjusting the position of the second detector 140 based on the location of the second region 106.

In some embodiments, the method 500 further comprises adjusting the position of the first radiation source 110 and/or the position of the second radiation source 120 based on the image 312 of the subject 102.

In some embodiments, the method 500 further comprises guiding, via the at least one movable mirror 410, the radiation from the subject 102 to the first detector 130 and/or the second detector 140. In some embodiments, the method 500 further comprises adjusting the position of the at least one movable mirror 410 based on the image 312 of the subject 102.

The present invention provides the improved system 100 and the corresponding method 500 for determining the physiological parameter 250 of the subject 102 with an increased accuracy. The system 100 and the method 500 utilize a combination of SV and PPG techniques in order to determine the PTT 230. The combination may eliminate or mitigate the respective disadvantages of the individual techniques while leveraging the unique properties of both the techniques. Specifically, the SV and PPG techniques are used at first and second regions 104, 106, respectively. The SV and PPG techniques used at first and second regions 104, 106, respectively, have synergetic properties for determination of the PTT 230. The system 100 of the present invention may be a completely contactless system and may provide a continuous measurement of the physiological parameter 250. Therefore, the system 100 may be suitable for the subject 102 with extreme skin sensitivity. The system 100 and the method 500 may provide a reliable assessment of the cardiovascular system of the subject 102. The system 100 and the method 500 may also be used for respiratory sleep monitoring of the subject 102.

There is thus provided a system and a method for determining a physiological parameter of a subject, which overcomes the existing problems.

There is also provided a computer program product comprising a computer readable medium having computer readable code embodied therein, the computer readable code being configured such that, on execution by a suitable computer or processing unit, the computer or processing unit is caused to perform the method or methods described herein.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub- routines. Many different ways of distributing the functionality among these sub-routines will be apparent to the skilled person. The sub-routines may be stored together in one executable file to form a self-contained program. Such an executable file may comprise computer- executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single processor, element or other unit may fulfill the functions of several items recited in the claims. The mere fact that certain measures are recited in mutually different dependent claims does not indicate that a combination of these measures cannot be used to advantage.

In the present disclosure, the expression "at least one of A, B and C" means "A, B, and/or C", and that it suffices if, for example, only B is present. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

List of Elements

| | |
|---|---|
| 100 | System |
| 102 | Subject |
| 104 | First Region |
| 103 | Mechanical Waves (MW) |
| 105 | Heart |
| 106 | Second Region |
| 107 | Cardiovascular Pressure Waves (CPW) |
| 108 | Skin |
| 109 | Physical Distance |
| 110 | First Radiation Source |
| 112 | Laser Radiation |
| 120 | Second Radiation Source |
| 122 | Electromagnetic Radiation |
| 124 | Positive Response |
| 130 | First Detector |
| 140 | Second Detector |

150 Processor
160 Bed
162 Blanket
170 Interface
172 Memory
210 First Image Data
212 Laser Speckle Pattern
214 SV-Signal
216 First Time
220 Second Image Data
222 Subject Radiation
224 PPG-Signal
226 Second Time
230 PTT
240 PWV
250 Physiological Parameter
301 Robotic Device
310 Overview Camera
312 Image
315 First Position
320 Chair
325 Second Position
330 Vertical Structure
340 First Robotic Arm
345 First Joint
350 Second Robotic Arm
355 Second Joint
360 First Position
370 Second Position
410 Movable Mirror
500 Method
502 Step
504 Step
506 Step
508 Step
510 Step
512 Step
514 Step

**Claims**

1. A system (100) for determining a physiological parameter (250) of a subject (102), the system (100) comprising:

      a first radiation source (110) configured to emit a laser radiation (112) towards a first region (104) of the subject (102) proximal to a heart (105) of the subject (102);
      a second radiation source (120) configured to emit an electromagnetic radiation (122) towards a second region (106) of the subject (102) remote from the first region (104) of the subject (102);
      a first detector (130) configured to acquire a first image data (210) from a laser speckle pattern (212) generated at the first region (104) in response to irradiation by the laser radiation (112) from the first radiation source (110);
a second detector (140) configured to acquire a second image data (220) from a subject radiation (222) reflected from or transmitted through a skin (108) at the second region (106) in response to irradiation by the electromagnetic radiation (122) from the second radiation source (120); and
a processor (150) for processing the first image data (210) and the second image data (220), wherein the processor (150) is communicably coupled to the first detector (130) and the second detector (140), and wherein the processor (150) is configured to:

      extract an SV-signal (214) from the first image data (210);
      extract a PPG-signal (224) from the second image data (220); and
      determine a PTT (230) based on the SV-signal (214) and the PPG-signal (224).

2. The system (100) of claim 1, wherein the processor (150) is further configured to:

      determine a first time (216) indicative of a systolic event at the first region (104) based on the SV-signal (214);
      determine a second time (226) indicative of an arrival of a cardiac pressure wave at the second region (106) based on the PPG-signal (224); and
      determine the PTT (230) based on a difference between the second time (226) and the first time (216).

3. The system (100) of claim 1 or 2, wherein the processor (150) is further configured to determine a PWV (240) based on the PTT (230) and a physical distance (109) between the first region (104) and the second region (106).

4. The system (100) of claim 3, wherein the processor (150) is further configured to determine the physiological parameter (250) based, at least in part, on the PWV (240) or the PTT (230).

5. The system (100) of any one of claims 1 to 4, wherein the first detector (130) is a camera, wherein the system (100) is configured to adjust a focus of the camera in order to acquire the first image data (210) from the laser speckle pattern (212).

6. The system (100) of any one of claims 1 to 5, further comprising an overview camera (310) configured to generate an image (312) of the subject (102).

7. The system (100) of claim 6, further configured to adjust a position of the first detector (130) and/or a position of the second detector (140) based on the image (312) of the subject (102).

8. The system (100) of claim 7, further configured to:

   determine a position (315, 325) of the subject (102) based on the image (312) of the subject (102); and
   adjust the position of the first detector (130) and/or the position of the second detector (140) based, at least in part, on the position (315, 325) of the subject (102).

9. The system (100) of claim 6 or 7, further configured to:

   determine a location of the first region (104) and a location of the second region (106) based on the image (312) of the subject (102);
   adjust the position of the first detector (130) based on the location of the first region (104); and
   adjust the position of the second detector (140) based on the location of the second region (106).

10. The system (100) of any one of claims 6 to 9, further configured to adjust a position of the first radiation source (110) and/or a position of the second radiation source (120) based on the image (312) of the subject (102).

11. The system (100) of claim 6, further comprising at least one movable mirror (330) configured to guide a radiation from the subject (102) to the first detector (130) and/or the second detector (140), and wherein the system (100) is further configured to adjust a position of the at least one movable mirror (330) based on the image (312) of the subject (102).

12. A method (500) for determining a physiological parameter (250) of a subject (102), the method (500) comprising:

   emitting a laser radiation (112) towards a first region (104) of the subject (102) proximal to a heart (105) of the subject (102);
   emitting an electromagnetic radiation (122) towards a second region (106) of the subject (102) remote from the first region (104) of the subject (102);
   acquiring a first image data (210) from a laser speckle pattern (212) generated at the first region (104) in response to irradiation by the laser radiation (112);
   acquiring a second image data (220) from a subject radiation (222) reflected from or transmitted through a skin (108) at the second region (106)

in response to irradiation by the electromagnetic radiation (122);

   extracting an SV-signal (214) from the first image data (210);
   extracting a PPG-signal (224) from the second image data (220); and
   determining a PTT (230) based on the SV-signal (214) and the PPG-signal (224).

13. The method (500) of claim 12, further comprising:

   determining a first time (216) indicative of a systolic event at the first region (104) based on the SV-signal (214);
   determining a second time (226) indicative of an arrival of a cardiac pressure wave at the second region (106) based on the PPG-signal (224); and
   determining the PTT (230) based on a difference between the second time (226) and the first time (216).

14. The method (500) of claim 12 or 13, further comprising:

   determining a physical distance (109) between the first region (104) and the second region (106);
   determining a PWV (240) based on the PTT (230) and the physical distance (109); and
   determining the physiological parameter (250) based, at least in part, on the PWV (240) or the PTT (230).

15. A computer program product containing computer readable code configured such that, on execution by a suitable computer or processing unit, the computer or processing unit is caused to perform the method (500) of any one of claims 12 to 14.

**FIG. 1**

EP 4 144 289 A1

FIG. 2

210

214

150

## FIG. 3A

220

224

150

## FIG. 3B

EP 4 144 289 A1

214

230

150

224

## FIG. 4

FIG. 5

230

240

150

109

**FIG. 6**

230

250

150

240

**FIG. 7**

FIG. 8A

EP 4 144 289 A1

FIG. 8B

310

312

150

FIG. 9

FIG. 10

EP 4 144 289 A1

*500*

```
┌─────────┐
│   502   │
└────┬────┘
     │
     ▼
┌─────────┐
│   504   │
└────┬────┘
     │
     ▼
┌─────────┐
│   506   │
└────┬────┘
     │
     ▼
┌─────────┐
│   508   │
└────┬────┘
     │
     ▼
┌─────────┐
│   510   │
└────┬────┘
     │
     ▼
┌─────────┐
│   512   │
└────┬────┘
     │
     ▼
┌─────────┐
│   514   │
└─────────┘
```

# FIG. 11

Europäisches
Patentamt

European
Patent Office

Office européen
des brevets

## EUROPEAN SEARCH REPORT

**Application Number**

EP 21 19 4655

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MATTHEW FARLEY ET AL: "Optical determination of cardiovascular health at a distance", PROCEEDINGS OF SPIE, vol. 7703, 5 April 2010 (2010-04-05), page 77030R, XP055344373, US DOI: 10.1117/12.853314 ISBN: 978-1-5106-1533-5 * the whole document * * in particular: * * sections 2.1, 2.2, 3.1, 3.3, 4.2 * ----- | 1-15 | INV. A61B5/00 A61B5/021 A61B5/024 A61B5/0285 A61B5/11 |
| X | WO 2020/110116 A1 (CONTINUSE BIOMETRICS LTD [IL]) 4 June 2020 (2020-06-04) * figures 1,2,5 * * page 2, lines 24-28 * * page 8, lines 9-12 * * page 10, lines 15-16 * * page 10, line 28 - page 11, line 21 * * page 11, lines 26-30 * * page 12, line 22 - page 13, line 17 * * page 13, line 31 - page 14, line 2 * * page 14, line 31 - page 17, line 8 * * page 17, line 22 - page 18, line 10 * ----- -/-- | 1-15 | **TECHNICAL FIELDS SEARCHED (IPC)** A61B |

The present search report has been drawn up for all claims

| Place of search | Date of completion of the search | Examiner |
|---|---|---|
| The Hague | 16 February 2022 | Albrecht, Ronald |

EPO FORM 1503 03.82 (P04C01)

**page 1 of 2**

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LE TAI ET AL: "Continuous Non-Invasive Blood Pressure Monitoring: A Methodological Review on Measurement Techniques", IEEE ACCESS, IEEE, USA, vol. 8, 24 November 2020 (2020-11-24), pages 212478-212498, XP011824012, DOI: 10.1109/ACCESS.2020.3040257 [retrieved on 2020-12-04] * the whole document * * in particular: * * figure 6f * * sections II.A.4, II.B.1 * * page 212480, left-hand column * ----- | 1-15 | |
| Y | GOLBERG MARK ET AL: "Large-scale clinical validation of noncontact and continuous extraction of blood pressure via multipoint defocused photonic imaging", APPLIED OPTICS, vol. 57, no. 7, 27 November 2017 (2017-11-27), page B45, XP055891008, US ISSN: 1559-128X, DOI: 10.1364/AO.57.000B45 * the whole document * * in particular: * * figures 3,4 * * sections 2.B, 3 * ----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 16 February 2022 | Albrecht, Ronald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT**
**ON EUROPEAN PATENT APPLICATION NO.**

EP 21 19 4655

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2022

| Patent document cited in search report | Publication date | Patent family member(s) | | Publication date |
|---|---|---|---|---|
| WO 2020110116 A1 | 04-06-2020 | EP | 3886686 A1 | 06-10-2021 |
| | | JP | 2022508237 A | 19-01-2022 |
| | | US | 2022039679 A1 | 10-02-2022 |
| | | WO | 2020110116 A1 | 04-06-2020 |

## REFERENCES CITED IN THE DESCRIPTION

**Patent documents cited in the description**

- WO 2015078735 A1

**Non-patent literature cited in the description**

- **MUEHLSTEFF et al.** Cuffless estimation of systolic blood pressure for short effort bicycle tests: the prominent role of the pre-ejection period. *International Conference of IEEE engineering and medicine,* 2006
- **VERKRUYSSE et al.** Remote plethysmographic imaging using ambient light. *Optics Express,* 22 December 2008, vol. 16 (26), 21434-21445
- **GOLBERG et al.** Large-scale clinical validation of noncontact and continuous extraction of blood pressure via multipoint defocused photonic imaging. *Applied Optics,* 01 March 2018, vol. 57 (7
- Cuffless blood pressure monitoring using PTT and PWV methods. **MISHRA ; BISWAJIT ; NILESH THAKKAR.** 2017 International Conference on Recent Innovations in Signal processing and Embedded Systems (RISE). IEEE, 2017